# EUROPEAN PATENT APPLICATION

(11) **EP 4 064 168 A1**
(43) Date of publication of application: **28.09.2022**
(21) Application number: 20813846.1
(22) Date of filing: 29.05.2020
(51) Int. Cl.: G06Q 40/08, A61B 5/00, G16H 50/20, G06N 20/00

(54) **ARTIFICIAL INTELLIGENCE INSURANCE SERVER AND ARTIFICIAL INTELLIGENCE INSURANCE SERVICE PROVIDING METHOD**

(30) Priority: 31.05.2019 KR 20190064873
(71) Applicant: Lunit Inc., Seoul 06241 (KR)
(72) Inventor: JEONG, Na Young, Seoul 06241 (KR); JANG, Min Hong, Seoul 06241 (KR)
(74) Representative: Grosse, Felix Christopher
(86) International application number: PCT/KR2020/007054
(87) International publication number: WO 2020/242269

(57) **Abstract**

Provided are an artificial intelligence insurance server and a method for providing an artificial intelligence insurance service. The artificial intelligence insurance server comprises a receiving unit for receiving input data including medical data of an insurance user, a comparison data selection unit for selecting comparison data based on the input data, and deriving first analysis data by analyzing the comparison data, an artificial intelligence analysis unit for obtaining second analysis data derived by performing artificial intelligence analysis on the input data, a comparison unit for comparing the first and the second analysis data and generating final analysis data using the first and the second analysis data and a decision-making unit for making at least one insurance-related decision using the final analysis data.

## Description

### TECHNICAL FIELD

The disclosure relates to an artificial intelligence insurance server and a method for providing an artificial intelligence insurance service.

### BACKGROUND

In the existing insurance industry, sales and marketing of insurance products are performed by collectively setting insurance products of interest by sex and age groups, then promoting them, and providing discounts through additional elements, such as subscription channels regardless of the characteristics of the insurance user or the coverage details of the products for the sale of insurance products.

Furthermore, the existing insurance subscription screening, *i*.*e*., underwriting, is carried out by considering demographic factors and factors causing accidents or diseases. However, these factors cannot be proven in many cases, and thus, it is inevitable to rely heavily on the notification statements of the insurance users, thereby having low credibility.

In addition, unlike the calculation of individual risks, subscription screening is performed based on a statistical basis of the entire population, and thus, there are cases where substantive individual risks are inaccurately determined.

Even from the perspective of the insurance companies, it is difficult to individually evaluate the insurance users, and each insurance company has difficulty in making its own differences, which makes it difficult to develop efficient marketing strategies.

Accordingly, demands are arising for inventions to obtain individual medical judgments using artificial intelligence, and provide differentiated insurance services for each insurance company based thereon.

### SUMMARY

Aspects of the disclosure provide an artificial intelligence insurance server that can precisely determine the individual risk of insurance users.

Aspects of the disclosure provide a method for providing an artificial intelligence insurance service that can precisely determine the individual risk of insurance users.

According to some aspects of the disclosure, an artificial intelligence insurance server includes a receiving unit for receiving input data including medical data of an insurance user, a comparison data selection unit for selecting comparison data based on the input data and deriving first analysis data by analyzing the comparison data, an artificial intelligence analysis unit for obtaining second analysis data derived by performing artificial intelligence analysis on the input data, a comparison unit for comparing the first and the second analysis data, and generating final analysis data using the first and the second analysis data and a decision-making unit for making at least one insurance-related decision using the final analysis data.

According to some aspects, the receiving unit determines whether the input data is sufficient, and when the input data is sufficient, transmits a progress signal to the comparison data selection unit.

According to some aspects, the medical data includes at least one of an X-ray, CT (Computed Tomography), a mammography image, an MRI (Magnetic Resonance Imaging) image, an ultrasound image, a pathology slide image, a tissue image, genomic data including multi-omics data, biological data, a medical report or personal health records (PHR).

According to some aspects, the comparison data includes cohort data of the insurance user selected from the input data.

According to some aspects, the input data includes at least one of demographic information, or information on factors causing accidents or diseases of the insurance user, and the cohort data is selected from at least one of the demographic information, or information on factors causing accidents or diseases.

According to some aspects, the cohort data is selected as data of a combination that is similar to the combination of the input data.

According to some aspects, the comparison data includes past data of the insurance user.

According to some aspects, the comparison data selection unit receives diagnosis data including diagnosis result of the input data, and converts the diagnosis data to first analysis data.

According to some aspects, the artificial intelligence analysis unit receives second analysis data for which artificial intelligence analysis is performed on the input data.

According to some aspects, the decision-making unit makes at least one decision on insurance product recommendation, underwriting or insurance premium discount calculation if the insurance user has not subscribed to insurance, and makes at least one decision on health care, insurance benefits payout or additional insurance product recommendation if the insurance user has subscribed to insurance.

According to some aspects, the decision-making unit makes at least one decision on insurance benefits payout, the comparison unit determines whether a first additional test is needed using the first analysis data and determines whether a second additional test is needed using the second analysis data, and the decision-making unit requests the insurance user for an additional test if at least one of the first or the second additional test is determined to be needed.

According to some aspects, the receiving unit receives whether an additional test is carried out from the insurance user, and transmits whether the additional test is carried out to the decision-making unit, and the decision-making unit reduces insurance benefits of diagnosis for further diagnosis of the insurance user, if the second additional test is determined to be needed and the additional test is not carried out.

According to some aspects, the receiving unit receives whether an additional test is carried out from the insurance user, and transmits whether the additional test is carried out to the decision-making unit, and the decision-making unit determines whether an early diagnosis is made if the first additional test is determined not to be needed, the second additional test is determined to be needed, and the additional test is carried out, and pays raised amount of insurance benefits if it is determined that the early diagnosis is made.

According to some aspects, the comparison data selection unit requests comparison data from a database, and the receiving unit receives the comparison data from the database and transmits the comparison data to the comparison data selection unit.

According to some aspects of the disclosure, a method for providing an artificial intelligence insurance service includes receiving input data including medical data of an insurance user, selecting comparison data based on the input data, obtaining first analysis data for the comparison data, obtaining second analysis data for the input data, comparison the first and the second analysis data, generating final analysis data using the first and the second analysis data, and making at least one decision using the final analysis data.

According to some aspects, the obtaining first analysis data for the comparison data includes obtaining first analysis data by performing artificial intelligence analysis on the comparison data.

According to some aspects, the obtaining first analysis data for the comparison data includes receiving diagnosis data of a medical staff for the comparison data, and obtaining the first analysis data by converting the diagnosis data.

According to some aspects, the input data includes at least one of demographic information, or information on factors causing accidents or diseases of the insurance user.

According to some aspects, the comparison data includes cohort data selected from at least one of the demographic information, or the information on factors causing accidents or diseases.

According to some aspects, the obtaining second analysis data includes selecting a corresponding portion of the input data that corresponds to the comparison data, and obtaining the second analysis data by analyzing the corresponding portion.

According to some aspects, the making at least one decision includes at least one of insurance product recommendation, underwriting or insurance premium discount calculation if the insurance user has not subscribed to insurance, and includes at least one of health care, insurance benefits payout or additional insurance product recommendation if the insurance user has subscribed to insurance.

According to some aspects, the making at least one decision includes insurance benefits payout, and the insurance benefits payout includes determining whether a first additional test is needed using the first analysis data, and determining whether a second additional test is needed using the second analysis data, and requesting the insurance user for an additional test if at least one of the first or the second additional test is determined to be needed.

According to some aspects, the insurance benefits payout includes reducing insurance benefits of diagnosis for further diagnosis of the insurance user if the second additional test is determined to be needed, and the additional test is not carried out.

According to some aspects, the insurance benefits payout includes determining whether an early diagnosis is made, if the first additional test is determined not to be needed, the second additional test is determined to be needed, and the additional test is carried out, and paying raised amount of insurance benefits if the early diagnosis is made.

According to some aspects of the disclosure, a method for providing an artificial intelligence insurance service includes obtaining input data including at least one medical data of an insurance user, performing artificial intelligence analysis on the medical data and providing information on at least one of underwriting, a recommended insurance product, a predicted insurance premium, or whether an additional test is needed for the insurance user based on the artificial intelligence analysis result.

According to some aspects, the input data includes at least one of demographic information, or information on factors causing accidents or diseases of the insurance user.

According to some aspects, the providing is providing whether an additional test is needed, and the providing whether an additional test is needed includes providing information on the additional test and information on disadvantages due to not implementing an additional test if the additional test is determined to be needed.

### BRIEF DESCRIPTION OF THE DRAWINGS

FIG. 1 is a conceptual diagram for illustrating an artificial intelligence insurance service system including an artificial intelligence insurance service according to some embodiments of the disclosure.
FIG. 2 is a block diagram for illustrating in detail the artificial intelligence insurance server of FIG. 1.
FIG. 3 is a conceptual diagram for illustrating in detail making at least one decision before and after obtaining insurance subscription performed by the decision-making unit of FIG. 2.
FIG. 4 is a table for illustrating a method for performing a procedure of insurance benefits payout by the decision-making unit of FIG. 2.
FIG. 5 is a conceptual diagram for illustrating an artificial intelligence insurance service system including an artificial intelligence insurance server according to some embodiments of the disclosure.
FIG. 6 is a block diagram for illustrating in detail the artificial intelligence insurance server of FIG. 5.
FIG. 7 is a conceptual diagram for illustrating an artificial intelligence insurance service system including an artificial intelligence insurance server according to some embodiments of the disclosure.
FIG. 8 is a flowchart for illustrating a method for providing an artificial intelligence insurance service according to some embodiments of the disclosure.
FIG. 9 is a flowchart for illustrating in detail obtaining second analysis data of FIG. 8.
FIG. 10 is a flowchart for illustrating in detail the insurance benefits payout during the decision-making of FIG. 8.
FIG. 11 is a flowchart illustrating a plurality of components for illustrating a method for providing an artificial intelligence insurance service according to some embodiments of the disclosure.
FIG. 12 is a flowchart illustrating a plurality of components for illustrating a method for providing an artificial intelligence insurance service according to some embodiments of the disclosure.
FIG. 13 is a flowchart illustrating a plurality of components for illustrating a method for providing an artificial intelligence insurance service according to some embodiments of the disclosure.
FIG. 14 is a flowchart illustrating a plurality of components for illustrating a method for providing an artificial intelligence insurance service according to some embodiments of the disclosure.
FIG. 15 is a flowchart illustrating a plurality of components for illustrating a method for providing an artificial intelligence insurance service according to some embodiments of the disclosure.
FIG. 16 is a flowchart illustrating a plurality of components for illustrating a method for providing an artificial intelligence insurance service according to some embodiments of the disclosure.
FIG. 17 is a flowchart illustrating a plurality of components for illustrating a method for providing an artificial intelligence insurance service according to some embodiments of the disclosure.
FIG. 18 is a block diagram of an electronic system for implementing an artificial intelligence insurance server and a method for providing an artificial intelligence insurance service according to some embodiments of the disclosure.

### DETAILED DESCRIPTION OF THE PREFERRED EMBODIMENTS

The advantages and features of the disclosed embodiments and methods of achieving them will be apparent when reference is made to the embodiments described below in conjunction with the accompanying drawings. However, the disclosure is not limited to the embodiments disclosed below, but may be implemented in various different forms. The disclosed embodiments are provided only to make the disclosure complete and are merely provided to fully convey the scope of the disclosure to those having ordinary skill in the art. The disclosure is defined only by the scope of the claims. The relative size of the regions and layers in the drawings m ay be exaggerated for clarify of description.

The same drawing reference numbers throughout the specification refer to the same component. The term "and/or" includes all combinations of each and one or more of the mentioned items.

Although the terms "first," "second," *etc.* are used to describe various elements, components and/or sections, these elements, components and/or sections are, of course, not limited by these terms. These terms are merely used to distinguish one element, component or section from another element, component or section. Accordingly, the first element, the first component or the first section mentioned below may, of course, refer to a second element, a second component or second section within the technical concept of the disclosure.

The terms used in the specification are for describing the embodiments and are not intended to limit the disclosure. In the specification, the singular form also includes the plural form unless clearly indicated in the context that it is singular. The terms "comprises" and/or "comprising" used in the specification do not indicate that the defined component, step, action and/or element exclude the existence or addition of one or more other component, step, action and/or element.

When a part is said to include "at least one of a, b or c", this means that the part may include only a, only b, only c, both a and b, both a and c, both b and c, all of a, b and c, or variations thereof.

Furthermore, the term "unit" used in the specification refers to software or hardware components, and a "unit" performs a certain role. However, this does not mean that a "unit" is limited to software or hardware. A "unit" may be configured to be in an addressable storage medium or configured to regenerate one or more processors. Accordingly, as an example, a "unit" may include components, such as software components, object-oriented software components, class components and task components, processors, functions, properties, procedures, sub-routines, segments of program codes, drivers, firmware, microcode, circuitry, data, database, data structures, tables, arrays and variables. The function provided within the components and "units" may be combined of a smaller number of components and "units" or further separated into additional components and "units."

Unless otherwise defined, all terms (including technical and scientific terms) used in the specification will be used as meanings that can be commonly understood by those of ordinary skill in the technical field to which the disclosure pertains. Furthermore, the terms defined in commonly used dictionaries are not interpreted ideally or exaggeratedly unless clearly and particularly defined.

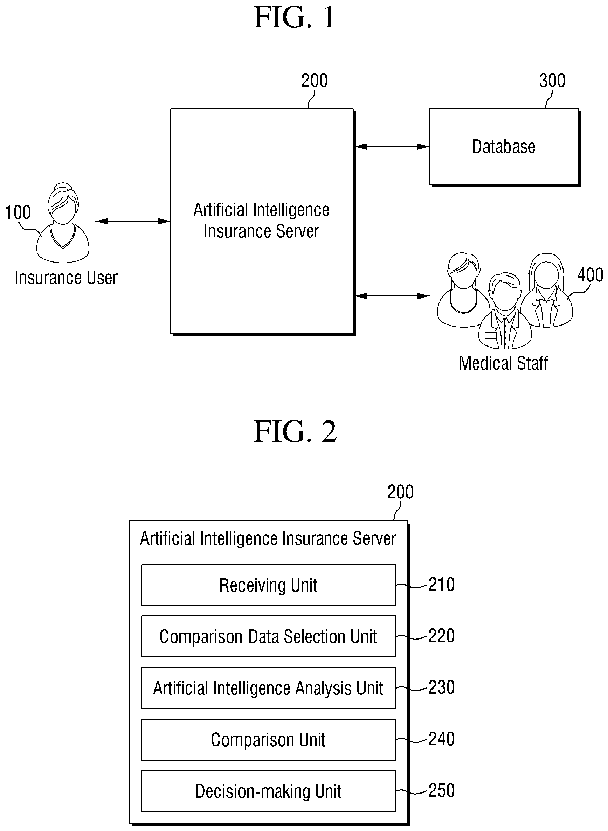

Below, an artificial intelligence insurance server according to an embodiment of the disclosure is described with reference to FIGS. 1 to 4.

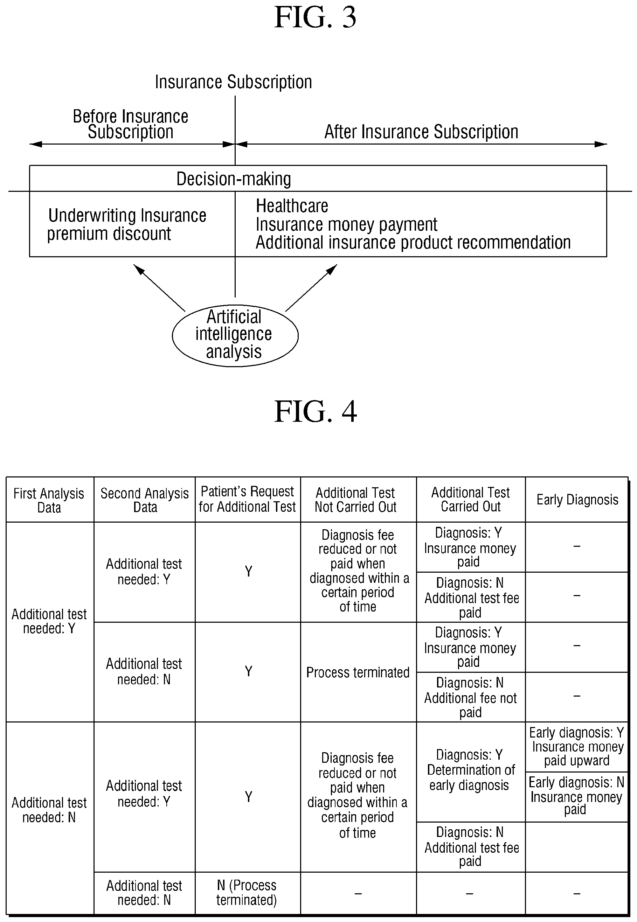

FIG. 1 is a conceptual diagram for illustrating an artificial intelligence insurance service system including an artificial intelligence insurance service according to some embodiments of the disclosure. FIG. 2 is a block diagram for illustrating in detail the artificial intelligence insurance server of FIG. 1. FIG. 3 is a conceptual diagram for illustrating in detail making at least one decision before and after obtaining insurance subscription performed by the decision-making unit of FIG.2. FIG. 4 is a table for illustrating a method for performing a procedure of insurance benefits payout by the decision-making unit of FIG. 2.

Referring to FIG. 1, an artificial intelligence insurance service system comprises an insurance user **100,** a first artificial intelligence insurance server **200,** a database **300** and a medical staff **400.**

The insurance user **100** may include both a person who intends to subscribe an insurance and a person that has already subscribed an insurance through the first artificial intelligence insurance server **200.** That is, the insurance user **100** may refer to anyone who is provided with insurance service through the first artificial intelligence insurance server **200.**

The insurance user **100** may transmit input data to the first artificial intelligence insurance server **200.** Here, the input data may include medical data of the insurance user **100,** demographic information of the insurance user **100** and information on factors causing accidents or diseases of the insurance user **100.** However, this embodiment is not limited thereto.

The medical data of the insurance user **100** may include various images of the body of the insurance user **100.** For example, the medical data may include an X-ray, CT (Computed Tomography), a mammography (DBT) image, an MRI (Magnetic Resonance Imaging) image, an ultrasound image and a pathology slide image. However, this embodiment is not limited thereto.

The demographic information of the insurance user **100** may include at least one of gender, age, height, weight or income of the insurance user **100.** However, this embodiment is not limited thereto.

Information on the factors causing accidents or diseases of the insurance user **100** may include at least one of occupation, whether they drive, whether they smoke, whether they drink or medical history of the insurance user **100.** However, this embodiment is not limited thereto.

There may be various methods in which the insurance user **100** transmits the input data to the first artificial intelligence insurance server **200.** For example, the insurance user **100** may upload the input data from a web page or an app page that can only be accessed by the insurance user **100.**

Alternatively, the insurance user **100** may simply give consent to providing the input data on the web page or the app page that can only be accessed by the insurance user **100,** and the first artificial intelligence insurance server **200** may pull up the input data from a medical institution, a medical artificial intelligence company, an insurance company, or the like. However, this embodiment is not limited thereto.

The first artificial intelligence insurance server **200** may provide the insurance user **100** with a service using artificial intelligence. The first artificial intelligence insurance server **200** may receive the input data from the insurance user **100.** After receiving the input data, the first artificial intelligence insurance server **200** may make various decisions by analyzing the input data using artificial intelligence.

Specifically, the making at least one decision may include at least one of underwriting, providing insurance premium discount information, health care, insurance benefits payout or additional insurance product recommendation. This is explained in further detail below.

The database **300** may provide various data to the first artificial intelligence insurance server **200.** The database **300** may retain, for example, cohort data for the input data and past data of the insurance user **100.** Based thereon, the database **300** may selectively provide the first artificial intelligence insurance server **200** with data requested by the first artificial intelligence insurance server **200.**

The medical staff **400,** as professional medical personnel, may receive data including medical data from the first artificial intelligence insurance server **200** and perform a diagnosis thereon. The medical staff **400** may transmit a diagnosis result, *i.e.,* diagnosis data, to the first artificial intelligence insurance server **200.**

FIG. 2 is a block diagram for illustrating in detail the artificial intelligence insurance server of FIG. 1.

Referring to FIGS. 1 and 2, the first artificial intelligence insurance server **200** includes a receiving unit **210,** a comparison data selection unit **220,** an artificial intelligence analysis unit **230,** a comparison unit **240** and a decision-making unit **250.**

The receiving unit **210** may receive all data received by the first artificial intelligence insurance server **200.** Specifically, the receiving unit **210** may receive the input data from the insurance user **100,** and receive comparison data from the database **300.** Furthermore, the receiving unit **210** may receive the diagnosis data from the medical staff **400.** However, this embodiment is not limited thereto, and parts other than the receiving unit **210** may receive data.

After receiving the input data from the insurance user **100,** the receiving unit **210** may determine whether the input data is sufficient. If the input data is sufficient, the receiving unit **210** may transmit a progress signal to the comparison data selection unit **220.** Through which, the comparison data selection unit **220** may perform subsequent actions.

If the input data is insufficient, the receiving unit **210** may wait without transmitting a progress signal to the comparison data selection unit **220.** In such case, the comparison data selection unit **220** does not perform a subsequent action without a progress signal, and thus, the procedure may be stopped.

When the receiving unit **210** determines whether the input data is sufficient, the determination may be made by considering both the qualitative factors and quantitative factors of the input data. That is, when the input data is insufficiently received, the receiving unit **210** may determine that it is insufficient. Furthermore, when the quality of the input data is poor, for example, if the data is a low-resolution image or partially damaged video data, the receiving unit **210** may determine that the input data is insufficient.

The receiving unit **210** may select data to be used for analysis while simultaneously determining whether the input data is sufficient. That is, if data that is not needed to be analyzed is included in the input data, an action that excludes this data may be performed.

The comparison data selection unit **220** may receive the input data from the receiving unit **210.** The comparison data selection unit **220** may select comparison data based on the input data.

Here, the comparison data may be cohort data of the input data. The cohort data may be selected from at least one of demographic information or information on factors causing accidents or diseases included in the input data.

Specifically, at least one of data including medical data of a subject similar to the demographic information of the insurance user **100,** data including medical data of a subject similar to the medical history of the insurance user **100** or data configured of a combination similar to the combination of the input data of the insurance user **100** may be selected as the cohort data. However, this embodiment is not limited thereto.

When the comparison data is selected as the cohort data, it may be easier to ascertain the individual characteristics of the insurance user **100** by comparing the comparison data with the input data of the insurance user **100.** Through which, the first artificial intelligence insurance server **200** may make more accurate decisions.

Alternatively, the comparison data may be data that was previously submitted by the insurance user **100.** In such case, the comparison data may be the data of the same type as the input data submitted by the insurance user **100,** or the data of a different type but a similar type according to an analysis.

Specifically, when the input data includes a chest X-ray image in which a lesion was photographed, the comparison data may include past chest X-ray images to enable a time-sequential comparison of the lesion.

Alternatively, when the input data includes a mammogram image, a time-sequential comparison with previously submitted mammogram images may be made.

When a time-sequential comparison between the comparison data and the input data may be made, the risk of the insurance user **100** may be accurately determined using the medical data of the same insurance user **100,** and thus, the accuracy of the decision-making of the first artificial intelligence insurance server **200** may be enhanced.

When the comparison data is the cohort data or the past data of the insurance user **100,** the comparison data selection unit **220** may request the database **300** for comparison data. Based thereon, the database **300** may transmit the requested data to the comparison data selection unit **220** via the receiving unit **210.**

The comparison data selection unit **220** may retain an index for retained data of the database **300,** or know the existence of a required scope in advance by requesting a search to the database **300.** Through which, the comparison data selection unit **220** may select the comparison data.

Alternatively, the comparison data may be the diagnosis data of the medical staff **400** for the input data submitted by the insurance user **100.** In such case, the comparison data selection unit **220** may transmit the input data to the medical staff **400.** The medical staff **400** may transmit the diagnosis data for the received input data to the receiving unit **210.**

The receiving unit **210** may transmit the diagnosis data to the comparison data selection unit **220,** and the comparison data selection unit **220** may convert the diagnosis data to generate first analysis data. The first analysis data may be data converted so that the diagnosis data may be compared in the same manner as second analysis data.

The artificial intelligence analysis unit **230** may include artificial intelligence for analyzing medical data therein. The artificial intelligence analysis unit **230** may generate second analysis data by analyzing the medical data included in the input data.

Specifically, the artificial intelligence analysis unit **230** may select a portion corresponding to the comparison data from the input data as a corresponding portion. Then, the artificial intelligence analysis unit **230** may perform artificial intelligent analysis on the corresponding portion to generate the second analysis data. Here, artificial intelligence analysis refers to an action deriving a diagnosis result by analyzing medical data using an artificial intelligence algorithm.

If the comparison data selection unit **220** selects the comparison data as the cohort data or the past data of the insurance user **100,** the artificial intelligence analysis unit **230** may perform artificial intelligence analysis on the comparison data to generate the first analysis data.

Alternatively, when the comparison data selection unit **220** has selected the comparison data as the cohort data or the past data of the insurance user **100,** the comparison data selection unit **220** may transmit the comparison data to the medical staff **400.** The medical staff **400** may generate diagnosis data for the comparison data after receiving the comparison data. The receiving unit **210** may receive the diagnosis data and redeliver this to the comparison data selection unit **220.** The comparison data selection unit **220** may convert the diagnosis data to generate the first analysis data.

As so, the first analysis data may be generated in various ways. That is, when the comparison data is the cohort data or the past data of the insurance user **100,** the first analysis data may be generated by artificial intelligence analysis or diagnosis of the medical staff **400.**

Alternatively, if the comparison data has been generated by the diagnosis of the medical staff **400,** the first analysis data may be generated by a simple conversion.

In contrast, the second analysis data may be generated by performing artificial intelligence analysis on the input data by the artificial intelligence analysis unit **230.**

The comparison unit **240** may compare the first analysis data with the second analysis data each other. The comparison unit **240** may record a value of comparison two analysis data displayed by different methods. The recorded value may later be used as learning data for the artificial intelligence analysis unit **230** or used in decision-making by being transmitted to the decision-making unit.

The comparison unit **240** may generate final analysis data using the first analysis data and the second analysis data. The final analysis data may be various forms of data derived from the first analysis data and the second analysis data.

The decision-making unit **250** may receive the final analysis data from the comparison unit **240.** The decision-making unit **250** may make at least one decision using the final analysis data.

FIG. 3 is a conceptual diagram for illustrating in detail making at least one decision before and after obtaining insurance subscription performed by the decision-making unit of FIG. 2.

Referring to FIGS. 2 and 3, the decision-making unit **250** may perform various decision-making processes according to the point of the insurance user **100** obtaining insurance subscription.

Specifically, the decision-making unit **250** may perform making at least one decision on insurance product recommendation, underwriting and insurance premium discount calculation if the insurance user **100** has not subscribed to insurance.

Insurance product recommendation may refer to making at least one decision to recommend which insurance product to take out before subscribed to insurance. The first artificial intelligence insurance server **200** may recommend an insurance product suitable to the risk of the insurance user **100** to encourage subscription.

Underwriting may refer to insurance subscription screening. That is, in order for the insurance user **100** to subscribe to an insurance, the user's current health state and health state expected in the future must be at least a specific level. Based thereon, when the health state of the insurance user **100** is precisely determined by analyzing each of the input data and the comparison data and comparing them with each other, the first artificial intelligence insurance server **200** may clearly determine whether the insurance user **100** is eligible to subscribe to the insurance.

Insurance premium discount may refer to making at least one decision to discount the insurance premium when the health state of the insurance user **100** is at least a specific level when subscribing to insurance. That is, if it is determined that the insurance user **100** is healthier than a general case and has a low risk, the first artificial intelligence insurance server **200** may promote the sale of an insurance product through a promotion of discounting the insurance premium of the insurance user **100.**

The decision-making unit **250** may transmit underwriting information and insurance premium discount information to the insurance user **100.** Through which, the insurance user **100** may clearly determine whether insurance subscription may be performed and the insurance premium may be discounted at the time of subscription through a precise risk determination based on artificial intelligence analysis. Through which, the insurance company connected to the artificial intelligence insurance server may suitably block the insurance subscription of the insurance user **100** with high risk, and suitably induce the insurance subscription of the insurance user **100** with low risk.

The decision-making unit **250** may make at least one decision on health care, insurance benefits payout and additional insurance product recommendation if the insurance user **100** has not subscribed to insurance.

Health care may refer to the first artificial intelligence insurance server **200** making at least one decision on recommending health care activities and associated services based on the health level and risk of the insurance user **100.** Through which, the insurance user **100** may check his or her own health level, and be suitably provided the required services. In addition, the first artificial intelligence insurance server **200** may obtain additional income by providing customized services needed for the insurance user **100.**

Here, associated services may include health examinations or additional tests. Accordingly, the first artificial intelligence insurance server **200** may provide the insurance user **100** with guidance on health examinations or suggestions on additional tests.

Insurance benefits payout among the decisions refers to making at least one decision on the insurance benefits payout as specified in the insurance product. Insurance benefits may include medical expenses, insurance benefits of diagnosis and insurance benefits of outpatient/inpatient treatment. Medical expenses refer to the actual amount of hospital fees, and insurance benefits of diagnosis refer to the insurance benefits payout made as a consolation benefit at the time of being diagnosed with a disease. Furthermore, insurance benefits of outpatient/inpatient treatment refer to the insurance benefits payout per case regardless of the actual amount of hospital fees.

Insurance benefits of diagnosis may vary depending on the type of disease, for example, 50 million won for cancer diagnosis and 5 million won for small claim cancer diagnosis, and may be set to a lower amount for early diagnosis. That is, small claim cancer may refer to the early stages of cancer. Insurance benefits of outpatient/inpatient treatment may be insurance benefits calculated by the time period regardless of the actual amount of hospital fees, such as '30,000 won/day of hospitalization.'

FIG. 4 is a table for illustrating a method for performing a procedure of insurance benefits payout by the decision-making unit of FIG. 2.

Referring to FIGS. 2 and 4, the decision-making unit **250** may determine whether first and second additional tests are needed using the first analysis data and the second analysis data. Here, whether the first and the second additional tests are needed may be one of the final analysis data generated based on a determination of the comparison unit **240.** That is, the decision-making unit **250** may receive whether the first and the second additional tests are needed as final analysis data. However, this embodiment is not limited thereto. The decision-making unit **250** may directly determine whether each of the first and the second additional tests are needed using the first analysis data and the second analysis data.

If both of the first and the second additional tests is determined not to be needed, the procedure may be terminated.

If any one of the first and the second additional tests is determined to be needed, the decision-making unit **250** may request the insurance user **100** for an additional test. Based thereon, the insurance user **100** may transmit whether an additional test is carried out to the decision-making unit **250** via the receiving unit **210.** If the insurance user **100** does not transmit whether the additional test is carried out by a certain period of time, it may be considered as transmitted that the additional test not being carried out to the decision-making unit **250.**

If the second additional test is determined not to be needed, and an additional test is not carried out, the procedure may be terminated.

If the second additional test is determined to be needed, and an additional test is not carried out, the decision-making unit **250** may reduce the insurance benefits of diagnosis when the insurance user **100** is diagnosed later within a certain period of time. Here, the reduction of the insurance benefits of diagnosis may be a concept that includes the non-payout of the insurance benefits of diagnosis. That is, the insurance benefits of diagnosis may be reduced in the form of a penalty when the insurance user **100** does not carried out an additional test despite the first artificial intelligence insurance server **200** having suggested the need for an additional test based on the second analysis data. This reflects that it may be determined that the reason attributable to the insurance user **100** is large.

If an additional test is carried out, the action of the decision-making unit **250** is determined based on whether a diagnosis is made. If a diagnosis is made, the decision-making unit **250** pays the insurance benefits, and if a diagnosis is not made, the decision-making unit **250** determines whether to pay an additional at expense.

If the first additional test is determined to be needed, but the second additional test is determined not to be needed, and the diagnosis is not made, the additional test expense may be unpaid. This may be due to the second analysis data that has performed artificial intelligence analysis on the input data of the insurance user **100** being more reliable than the first analysis data.

If the first additional test is determined not to be needed and the second additional test is determined to be needed, and the diagnosis is made, the decision-making unit **250** determines whether an early diagnosis is made. If an early diagnosis is not made, the decision-making unit **250** pays the insurance benefits as is, and if an early diagnosis is made, the decision-making unit **250** may pay raised amount of the insurance benefits. This may be due to the second analysis data being more reliable than the first analysis data as a reward of early diagnosis based on the second analysis data.

Again, referring to FIGS. 2 and 3, additional insurance product recommendation from the decisions made refers to making at least one decision on recommending a different insurance product other than the insurance product to which the insurance user **100** currently subscribes.

That is, when abnormal symptoms of a disease other than that covered by the currently subscribed insurance product during the process of analyzing the medical data of the insurance user **100,** the decision-making unit **250** may provide a recommendation of another insurance product to the insurance user **100.** Through which, the insurance user **100** may identify currently needed insurance products, and the insurance company connected to the first artificial intelligence insurance server **200** may gain profit therefrom.

The first artificial intelligence insurance server **200** according to the embodiment may efficiently make at least one decision by precisely determining the health state and risk of the insurance user **100** using artificial intelligence. Furthermore, in terms of the insurance benefits payout, the first artificial intelligence insurance server **200** according to the embodiment may provide the insurance user **100** with penalties and rewards based on information in the first and the second analysis data, so that efficient management of the insurance benefits may be achieved.

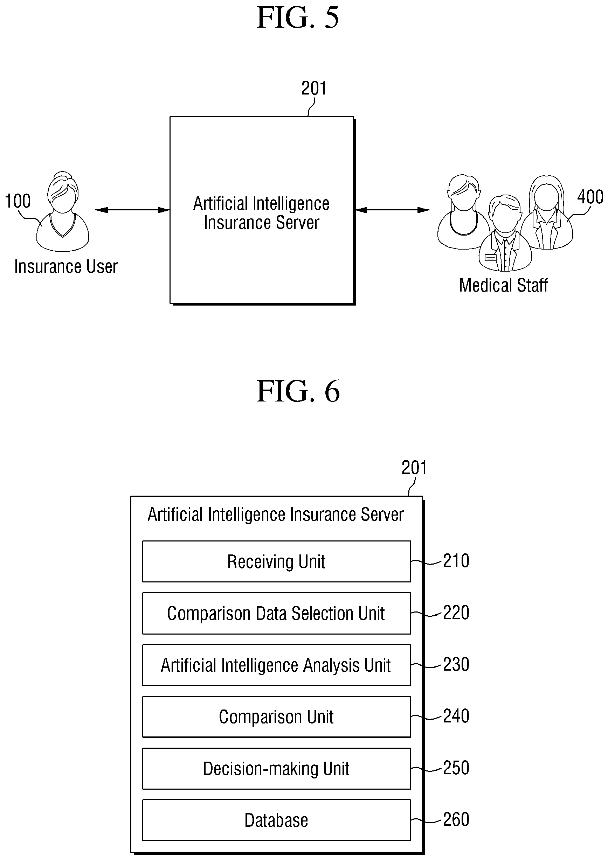

Below is a description of the artificial intelligence insurance server according to some embodiments of the disclosure in reference to FIGS. 5 and 6. Any parts that overlap with the descriptions above are simplified or omitted.

FIG. 5 is a conceptual diagram for illustrating an artificial intelligence insurance service system including an artificial intelligence insurance server according to some embodiments of the disclosure. FIG. 6 is a block diagram for illustrating in detail the artificial intelligence insurance server of FIG. 5.

Referring to FIGS. 5 and 6, the second artificial intelligence insurance server 201 according to some embodiments of the disclosure may include a database **260** therein. Based thereon, even if the comparison data is the cohort data or the past data of the insurance user **100,** there is no need for the receiving unit **210** to receive the comparison data, and the database **260** may directly transmit the comparison data to the comparison data selection unit **220.**

The second artificial intelligence insurance server **201** according to the embodiment retains a database **260** therein, so that the data may be efficiently transmitted. In addition, the comparison data selection unit **220** may easily determine the data retained by the database **300** during the process of selecting the comparison data, so that more rapid and efficient decisions may be made.

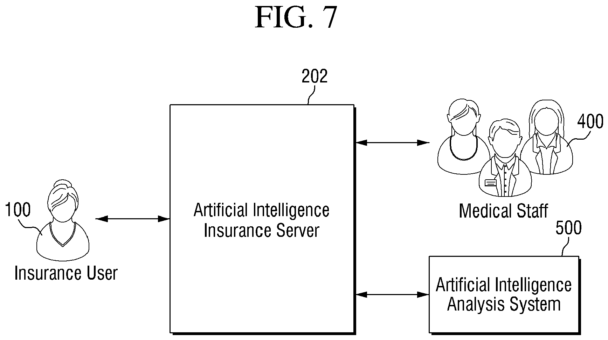

Below is a description of the artificial intelligence insurance server according to some embodiments of the disclosure in reference to FIG. 7. Any parts that overlap with the descriptions above are simplified or omitted.

FIG. 7 is a conceptual diagram for illustrating an artificial intelligence insurance service system including an artificial intelligence insurance server according to some embodiments of the disclosure.

Referring to FIG. 7, the artificial intelligence insurance service system including a third artificial intelligence insurance server **202** according to some embodiments of the disclosure further comprises an artificial intelligence analysis system **500.**

The artificial intelligence analysis system **500** may receive data from the third artificial intelligence insurance server **202** to perform artificial intelligence analysis. The artificial intelligence analysis system **500** may transmit the data that is performed artificial intelligence analysis to the third artificial intelligence insurance server **202** again.

Specifically, the third artificial intelligence insurance server **202** may transmit the input data to the artificial intelligence analysis system **500.** The artificial intelligence analysis system **500** may perform artificial intelligence analysis on the input data to generate the second analysis data, and may transmit it to the third artificial intelligence insurance server **202** again.

Furthermore, when the comparison data is selected as the cohort data or the past data of the insurance user **100,** the third artificial intelligence insurance server **202** may transmit the comparison data to the artificial intelligence analysis system **500,** and the artificial intelligence analysis system **500** may perform artificial intelligence analysis on the comparison data to generate the first analysis data. The artificial intelligence analysis system **500** may transmit the first analysis data to the third artificial intelligence insurance server **202.**

The description above has explained that all artificial intelligence analysis is performed by the artificial intelligence analysis system **500.** However, this embodiment is not limited thereto. That is, the third artificial intelligence insurance server **202** according to the embodiment may directly perform some artificial intelligence analysis, and the remaining artificial intelligence analysis may be performed and received through the artificial intelligence analysis system **500.**

The third artificial intelligence insurance server **202** according to the embodiment includes at least one module for artificial intelligence analysis on the outside, so that resources needed in other actions, such as decision-making, may be carried out without deficit. Based thereon, the third artificial intelligence insurance server **202** may secure a precise result according to artificial intelligence analysis and simultaneously make efficient decisions.

Below is a description of a method for providing an artificial intelligence insurance service according to some embodiments of the disclosure in reference to FIGS. 1 to 4 and FIGS. 8 to 10. Any parts that overlap with the descriptions above are simplified or omitted.

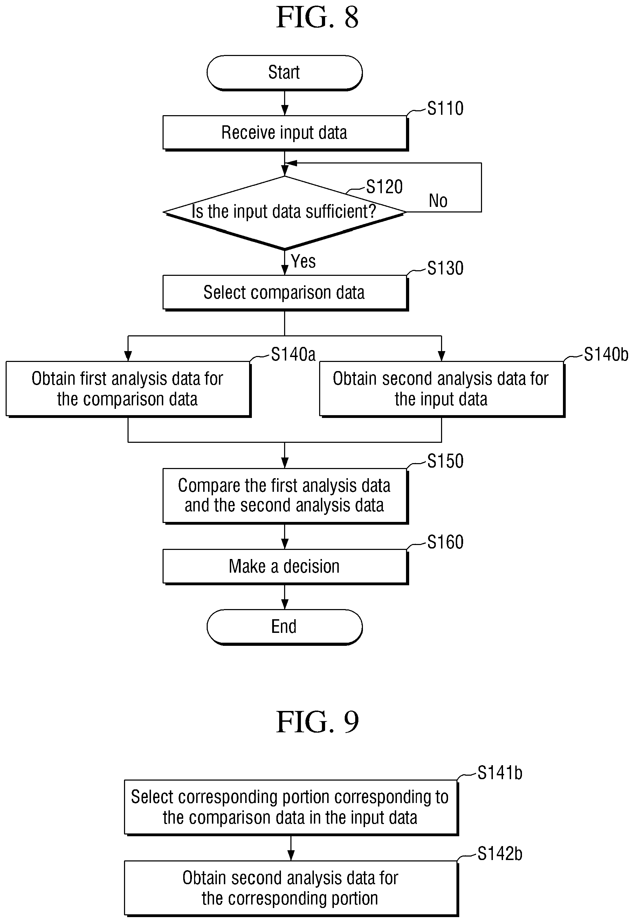

FIG. 8 is a flowchart for illustrating a method for providing an artificial intelligence insurance service according to some embodiments of the disclosure. FIG. 9 is a flowchart for illustrating in detail obtaining second analysis data of FIG. 8.

Referring to FIG. 8, input data is received **S110.**

Specifically, referring to FIG. 1, the input data may include the medical data of the insurance user **100,** the demographic information of the insurance user **100** and the information on factors causing accidents and diseases of the insurance user **100.** However, this embodiment is not limited thereto.

Again, referring to FIG. 8, it is determined whether input data is sufficient **S120.**

Specifically, referring to FIG. 2, the receiving unit **210** may determine whether the input data is sufficient. When the input data is sufficient, the receiving unit **210** may transmit a progress signal to the comparison data selection unit **220,** and when the input data is insufficient, the receiving unit **210** may wait without transmitting a progress signal to the comparison data selection unit **220.** The receiving unit **210** determining whether the input data is sufficient may be determined by considering both the qualitative factors and the quantitative factors of the input data.

Again, referring to FIG. 8, comparison data is selected **S130.**

Specifically, referring to FIG. 2, the comparison data selection unit **220** may select the comparison data based on the input data. Here, the comparison data may be the cohort data of the input data or the data previously submitted by the insurance user **100.**

When the comparison data is the cohort data or the past data of the insurance user **100,** the comparison data selection unit **220** may request the database **300** for the comparison data. Based thereon, the database **300** may transmit the requested data to the comparison data selection unit **220** via the receiving unit **210.**

Alternatively, the comparison data may be the diagnosis data of the medical staff **400** regarding the input data submitted by the insurance user **100.** In such case, the comparison data selection unit **220** may transmit the input data to the medical staff **400.** The medical staff **400** may transmit the diagnosis data regarding the received input data to the receiving unit **210.** Again, referring to FIG. 8, first analysis data for the comparison data is obtained **S140a.**

Specifically, referring to FIG. 2, the first analysis data may be generated in various ways. That is, when the comparison data is the cohort data or the past data of the insurance user **100,** the first analysis data may be generated by artificial intelligence analysis or a diagnosis of the medical staff **400.** Alternatively, if the comparison data was generated by the diagnosis of the medical staff **400,** the first analysis data may be generated by a simple conversion.

Again, referring to FIG. 8, second analysis data for input data is obtained **S140b.**

Specifically, referring to FIG. 2, the artificial intelligence analysis unit **230** may analyze the medical data included in the input data to generate the second analysis data.

Referring to FIG. 9, a corresponding portion of the input data that corresponds to the comparison data is selected **S141b.** Subsequently, second analysis data for the corresponding portion is obtained **S142b.**

Again, referring to FIG. 8, first analysis data and second analysis data are compared **S150.**

Specifically, referring to FIG. 2, the comparison unit **240** may compare the first analysis data and the second analysis data with each other. The comparison unit **240** may generate final analysis data using the first analysis data and the second analysis data. The final analysis data may be various forms of data derived from the first analysis data and the second analysis data.

Again, referring to FIG. 8, making at least one decision is performed **S160.**

Specifically, referring to FIGS. 2 and 3, the decision-making unit **250** may make at least one decision on underwriting or insurance premium discount if the insurance user has not subscribed to insurance. The decision-making unit **250** may make at least one decision on health care, insurance benefits payout or additional insurance product recommendation if the insurance user has subscribed to insurance.

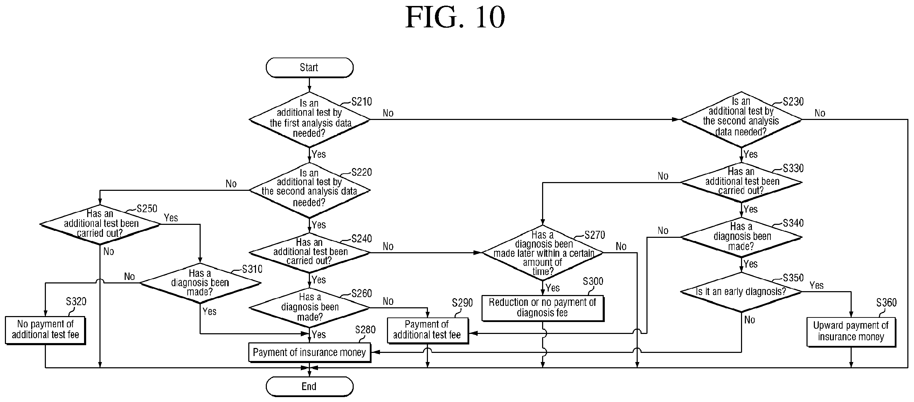

FIG. 10 is a flowchart for illustrating in detail the insurance benefits payout during the decision-making of FIG. 8.

Referring to FIGS. 4 and 10, it is determined whether an additional test is needed based on first analysis data **S210.** If an additional test is needed, the process proceeds to step **S220,** and if not, the process proceeds to step **S230.**

If an additional test is needed, it is determined whether an additional test is needed based on second analysis data **S220.** If an additional test is needed, the process proceeds to step **S240,** and if not, the process proceeds to step **S250.**

All results of step **S210** and step **S220** may be notified to the insurance user **100.**

If an additional test is needed, it is determined whether an additional test is carried out **S240.** Here, whether an additional test is carried out may be received from the insurance user **100.** If an additional test is carried out, the process proceeds to step **S260,** and if not, the process proceeds to step **S270.**

If an additional test is carried out, it is determined whether a diagnosis is made **S260.** Here, whether a diagnosis is made may be checked by receiving the result of the additional test. If a diagnosis is made, the insurance benefits is paid **S280,** and if not, an additional test expense is paid **S290.** The reason that the additional test expense is paid if a diagnosis has not been made is because the insurance user **100** has been notified that an additional test is needed based on the second analysis data, so the insurance user **100** is not required to personally cover the additional test expense.

If the additional test is not carried out in step **S240,** it is determined whether a diagnosis is made later within a certain period of time **S270.** If so, the insurance benefits of diagnosis are reduced or not paid **S300.** If not, the process is terminated.

Reducing or not paying the insurance benefits of diagnosis when a diagnosis is made may be a penalty for the insurance user **100** that does not carry out an additional test despite being notified that an additional test is needed based on the second analysis data.

If an additional test is not needed in step **S220,** it is determined whether an additional test is carried out **S250.** If so, the process proceeds to step **S310,** and if not, the process is terminated.

If an additional test is carried out, it is determined whether a diagnosis is made **S310.** If so, the insurance benefits are paid **S280,** and if not, the additional test expense is not paid **S320.**

This is because it is notified that an additional test is not needed based on the second analysis data, and thus, there is no need for the insurance user **100** to pay the additional test expense.

If an additional test is not needed in step **S210,** it is determined whether an additional test is needed based on the second analysis data **S230.** If so, the process proceeds to step **S330,** and if not, the process is terminated.

If an additional test is needed, it is determined whether an additional test is carried out **S330.** Here, whether an additional test is carried out may be received from the insurance user **100.** If an additional test is carried out, the process proceeds to step **S340,** and if not, the process proceeds to step **S270.**

If an additional test is carried out, it is determined whether a diagnosis is made **S340.** If a diagnosis is made, the process proceeds to step **S350,** and if not, the process proceeds to step **S290.**

If a diagnosis is made, it is determined whether an early diagnosis is made **S350.** If so, raised amount of the insurance benefits are paid **S360,** and if not, the insurance benefits are paid **S280.**

This is because when an early diagnosis is made, the disease is discovered early based on the second analysis data, and thus, may be a concept of providing the insurance user **100** with a reward based on the notification statement. Furthermore, in the case of an early diagnosis, the total sum of the insurance benefits may be decreased, and thus, it is possible for the insurance company connected to the first artificial intelligence insurance server **200** may obtain benefits.

If a diagnosis is not made in step **S340,** an additional test expense is paid **S290.** This is because an additional test was carried out according to the determination that an additional test is needed based on the second analysis data regardless of the determination based on the first analysis data. That is, the method for providing an artificial intelligence insurance service according to the embodiment uses that the reliability of the determination based on the second analysis data is higher than the reliability of the determination based on the first analysis data.

If an additional test is not carried out in step **S330,** it is determined whether a diagnosis is made later within a certain period of time **S270.** If so, the insurance benefits of diagnosis are reduced or not paid **S300.** If not, the process is terminated.

Reducing or not paying the insurance benefits of diagnosis when a diagnosis is made may be a penalty for the insurance user **100** that does not carry out an additional test despite being notified that an additional test is needed based on the second analysis data.

The method for providing an artificial intelligence insurance service according to the embodiment may efficiently perform the insurance benefits payout using artificial intelligence analysis data to induce a lower expenditure than the existing expenditure. In addition, it promotes early diagnosis, which may increase the health of the insurance user **100** to a high level and at the same time, lower the expenditure of insurance premium.

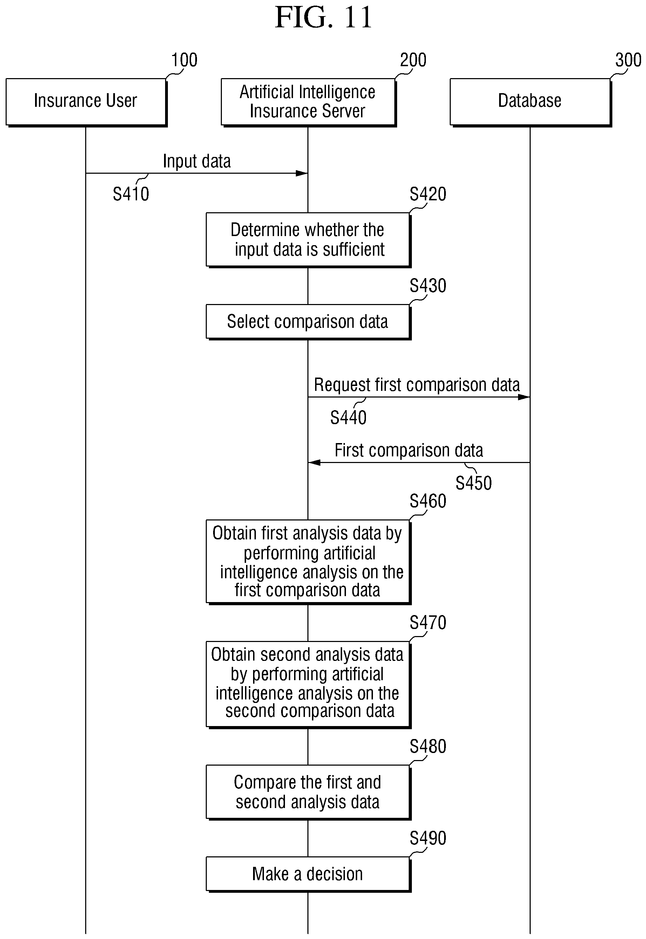

Below is a description of the method for providing an artificial intelligence insurance service according to some embodiments of the disclosure in reference to FIGS. 1 and 11. Any parts that overlap with the descriptions above are simplified or omitted.

FIG. 11 is a flowchart illustrating a plurality of components for illustrating a method for providing an artificial intelligence insurance service according to some embodiments of the disclosure.

Referring to FIGS. 1 and 11, the insurance user **100** transmits the input data to the first artificial intelligence insurance server **200.**

Then, the first artificial intelligence insurance server **200** determines whether the input data is sufficient **S420.** Then, the first artificial intelligence insurance server **200** selects the comparison data **S430.** Here, the comparison data may be selected as first comparison data including the cohort data or the past data of the insurance user **100.**

Then, the first artificial intelligence insurance server **200** requests the database **300** for first comparison data **S440.** The database **300** transmits the first comparison data to the first artificial intelligence insurance server **200, S450.**

Then, the first artificial intelligence insurance server **200** obtains fist analysis data by performing artificial intelligence analysis on the first comparison data **S460.** Then, the first artificial intelligence insurance server **200** obtains second analysis data by performing artificial intelligence analysis on the input data **S470.**

In FIG. 11, step **S460** and step **S470** are depicted to be consecutively performed. However, this embodiment is not limited thereto. That is, step **S460** and step **S470** may be performed in an opposite order, and may be performed in parallel.

Subsequently, the first artificial intelligence insurance server **200** performs a comparison on the first and the second analysis data **S480.** The first artificial intelligence insurance server **200** uses this to derive the final analysis data, and make at least one decision **S490.**

The method for providing an artificial intelligence insurance service according to the embodiment includes a database **300** on the outside, and thus, that the first artificial intelligence insurance server **200** does not need to use resources in data storage, so that more rapid and accurate decisions may be made. In addition, since the first comparison data and the second comparison data both perform artificial intelligence analysis, the first analysis data and the second analysis data are similar in form, thereby allowing easy comparison to each other and the efficiency of deriving the final analysis data may be enhanced.

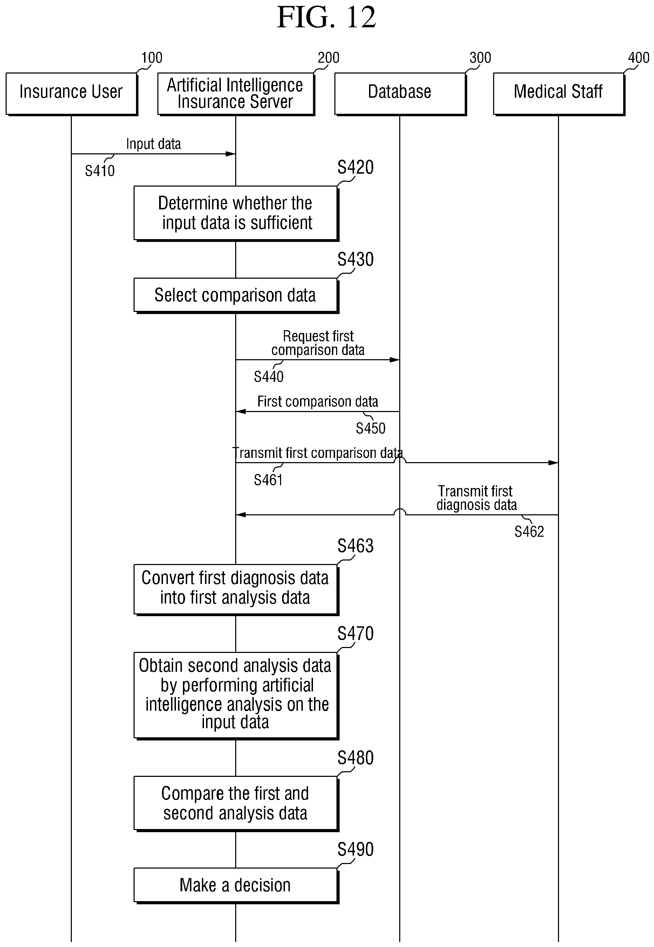

Below is a description of the method for providing an artificial intelligence insurance service according to some embodiments of the disclosure in reference to FIGS. 1 and 12. Any parts that overlap with the descriptions above are simplified or omitted.

FIG. 12 is a flowchart illustrating a plurality of components for illustrating a method for providing an artificial intelligence insurance service according to some embodiments of the disclosure.

Referring to FIGS. 1 and 12, step **S410** to step **S450** are identical to the description of FIG. 11. Subsequently, the first artificial intelligence insurance server **200** transmits the first comparison data to the medical staff **400, S461.** The medical staff **400** transmits the first diagnosis data, which is the diagnosis result for the first comparison data, to the first artificial intelligence insurance server **200** again **S462.**

Then, the first artificial intelligence insurance server **200** converts the first diagnosis data to first analysis data **S463.** A description of step **S470** to step **S490** hereinafter is identical to the description of FIG. 11

The method for providing an artificial intelligence insurance service according to the embodiment may supplement specific determinations that may be insufficient in the artificial intelligence analysis by converting the diagnosis result of the medical staff for the first comparison data to generate the first analysis data. Through which, the method for providing an artificial intelligence insurance service according to the embodiment may make more accurate and efficient decisions.

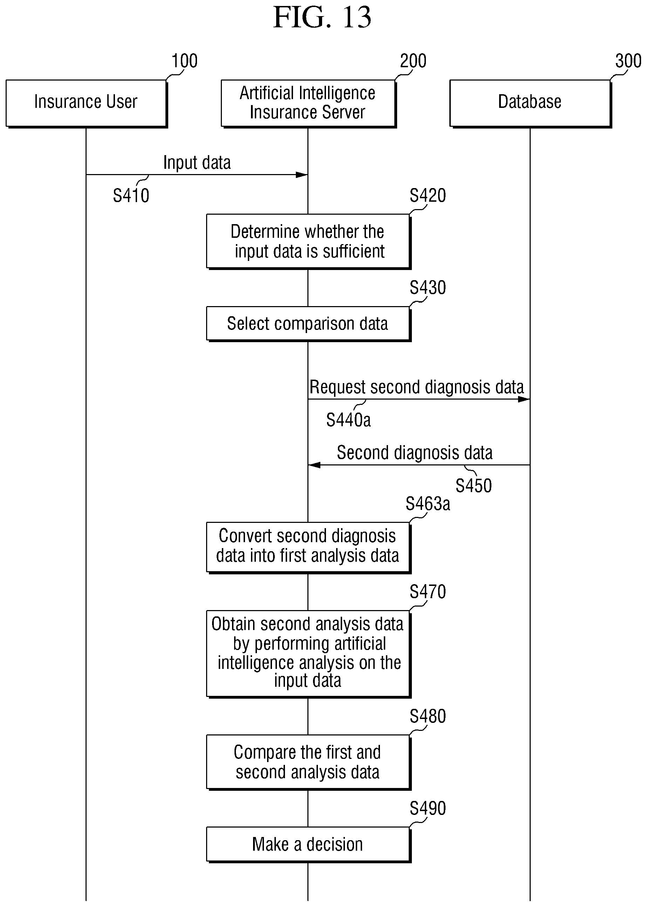

Below is a description of the method for providing an artificial intelligence insurance service according to some embodiments of the disclosure in reference to FIGS. 1 and 13. Any parts that overlap with the descriptions above are simplified or omitted.

FIG. 13 is a flowchart illustrating a plurality of components for illustrating a method for providing an artificial intelligence insurance service according to some embodiments of the disclosure.

Referring to FIGS. 1 and 13, step **S410** and step **S420** are identical to the description of FIG. 11. Subsequently, the first artificial intelligence insurance server **200** selects the comparison data **S430.** Here, the comparison data may be selected as second diagnosis data, which is the diagnosis result of the medical staff **400** for the input data of the insurance user **100.**

Then, the first artificial intelligence insurance server **200** requests the medical staff **400** for second diagnosis data **S440a.** The medical staff **400** transmits the second diagnosis data to the first artificial intelligence insurance server **200 S450a.**

Then, the first artificial intelligence insurance server **200** converts the second diagnosis data to first analysis data **S463a.** A description of step **S470** to step **S490** hereinafter is identical to the description of FIG. 11.

The method for providing an artificial intelligence insurance service according to the embodiment sets the comparison data itself as second diagnosis data, which is the diagnosis result of the medical staff **400** for the input data, to generate the first analysis data. Through which, the analysis of the medical staff **400** and the artificial intelligence analysis on the same input data are compared, thereby supplementing the disadvantages that each method may have to generate more precise analysis data. Through which, the method for providing an artificial intelligence insurance service according to the embodiment may make more accurate and efficient decisions.

Below is a description of the method for providing an artificial intelligence insurance service according to some embodiments of the disclosure in reference to FIGS. 5, 6 and 14. Any parts that overlap with the descriptions above are simplified or omitted.

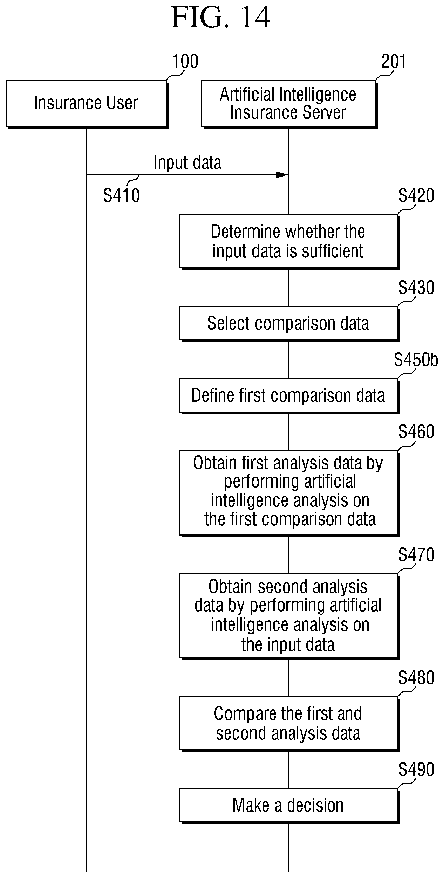

FIG. 14 is a flowchart illustrating a plurality of components for illustrating a method for providing an artificial intelligence insurance service according to some embodiments of the disclosure.

Referring to FIGS. 5, 6 and 14, a description of step **S410** to step **S430** is identical to the description of FIG. 11. Subsequently, the second artificial intelligence insurance server **201** defines the first comparison data **S450b.** That is, since the second artificial intelligence insurance server **201** includes a database **260** therein, it can define the first comparison data from therein. A description of step **S460** to step **S490** hereinafter is identical to the description of FIG. 11.

The method for providing an artificial intelligence insurance service according to the embodiment retains a database **260** within the second artificial intelligence insurance server **201,** so that data may be efficiently transmitted. In addition, the comparison data selection unit **220** can easily determine the data retained by the database **300** in the process of selecting the comparison data, and thus, more rapid and efficient decisions may be made.

Below is a description of the method for providing an artificial intelligence insurance service according to some embodiments of the disclosure in reference to FIGS. 5, 6 and 15. Any parts that overlap with the descriptions above are simplified or omitted.

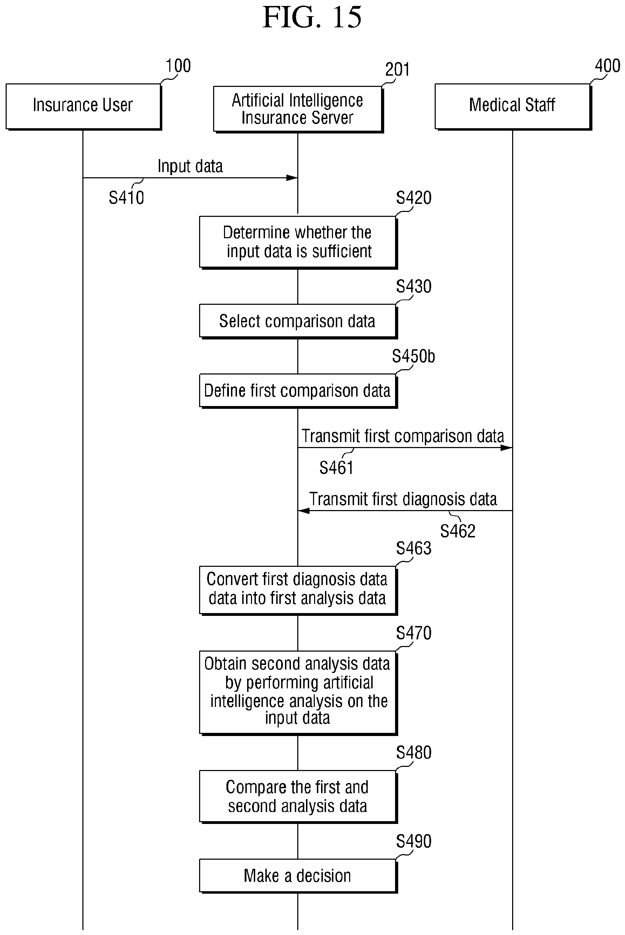

FIG. 15 is a flowchart illustrating a plurality of components for illustrating a method for providing an artificial intelligence insurance service according to some embodiments of the disclosure.

Referring to FIGS. 5, 6 and 15, a description of step **S410** to step **S430** is identical to the description of FIG. 11. Subsequently, the second artificial intelligence insurance server **201** transmits the first comparison data to the medical staff **400 S461.** The medical staff **400** transmits the first diagnosis data, which is the diagnosis result of the first comparison data, to the second artificial intelligence insurance server **201 S462.** Then, the second artificial intelligence insurance server **201** converts the first diagnosis data to first analysis data **S463.** A description of step **S470** to step **S490** hereinafter is identical to the description of FIG. 11.

The method for providing an artificial intelligence insurance service according to the embodiment may supplement specific determinations that may be insufficient in the artificial intelligence analysis by converting the diagnosis result of the medical staff regarding the first comparison data to generate the first analysis data. Through which, the method for providing an artificial intelligence insurance service according to the embodiment may make more accurate and efficient decisions.

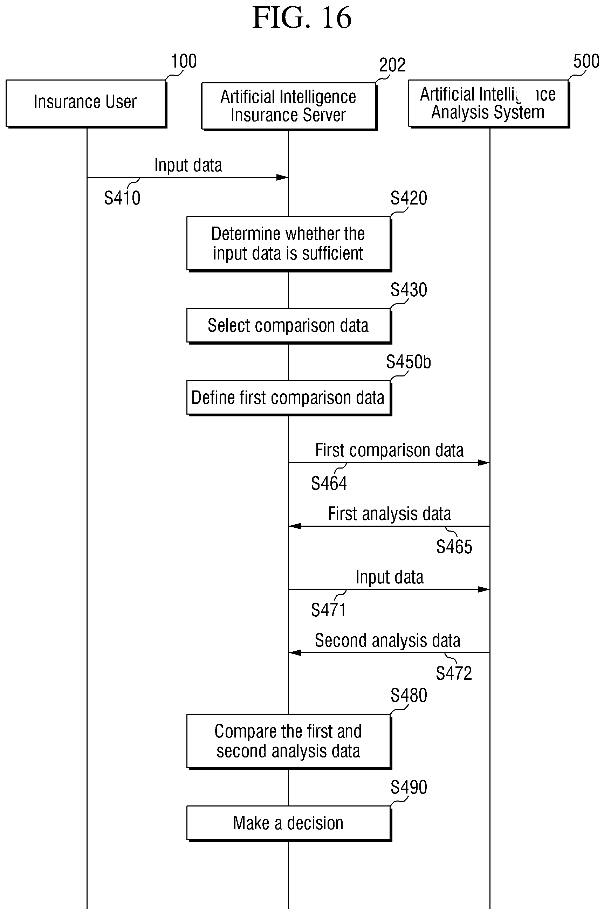

Below is a description of the method for providing an artificial intelligence insurance service according to some embodiments of the disclosure in reference to FIGS. 7 and 16. Any parts that overlap with the descriptions above are simplified or omitted.

FIG. 16 is a flowchart illustrating a plurality of components for illustrating a method for providing an artificial intelligence insurance service according to some embodiments of the disclosure.

Referring to FIGS. 7 and 16, a description of step **S410** to step **S430** is identical to the description of FIG. 11. A description of step **S450b** is identical to the description of FIG. 14.

Subsequently, the third artificial intelligence insurance server **202** transmits the first comparison data to the artificial intelligence analysis system **500 S464.** The artificial intelligence analysis system **500** transmits the first analysis data, which is an artificial intelligence analysis result of the first comparison data, to the third artificial intelligence insurance server **202, S465.** Then, the third artificial intelligence insurance server **202** transmits the input data to the artificial intelligence analysis system **500, S471.** The artificial intelligence analysis system **500** transmits the second analysis data, which is an artificial intelligence analysis result of the input data, to the third artificial intelligence insurance server **202, S472.**

A description of step **S480** to step **S490** hereinafter is identical to the description of FIG. 11.

The third artificial intelligence insurance server **202** of the method for providing an artificial intelligence insurance service according to the embodiment includes at least one module for artificial intelligence analysis on the outside, so that resources needed in other actions, such as decision-making, may be carried out without deficit. Based thereon, the third artificial intelligence insurance server **202** may secure a precise result according to artificial intelligence analysis and simultaneously make efficient decisions.

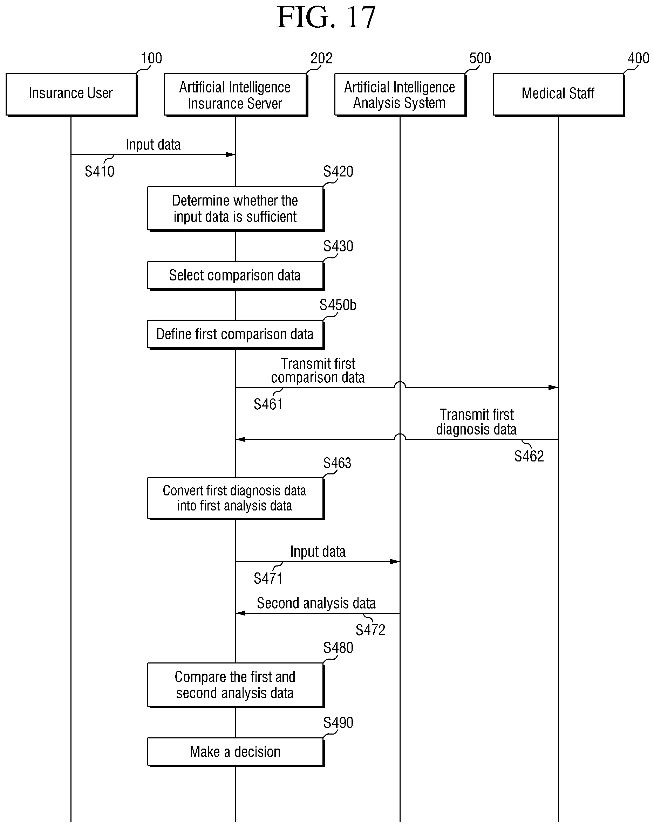

Below is a description of the method for providing an artificial intelligence insurance service according to some embodiments of the disclosure in reference to FIGS. 7 and 17. Any parts that overlap with the descriptions above are simplified or omitted.

FIG. 17 is a flowchart illustrating a plurality of components for illustrating a method for providing an artificial intelligence insurance service according to some embodiments of the disclosure.

Referring to FIGS. 7 and 17, a description of step **S410** to step **S430** is identical to the description of FIG. 11. A description of step **S450b** is identical to the description of FIG. 14. A description of step **S461** to step **S463** is identical to the description of FIG. 15. A description of step **S471** and step **S472** is identical to the description of FIG. 16. A description of step **S480** and step **S490** hereinafter is identical to the description of FIG. 11.

The method for providing an artificial intelligence insurance service according to the embodiment may supplement specific determinations that may be insufficient in the artificial intelligence analysis by converting the diagnosis result of the medical staff for the first comparison data to generate the first analysis data. Through which, the method for providing an artificial intelligence insurance service according to the embodiments may make more accurate and efficient decisions.

The method for providing an artificial intelligence insurance service described above may be implemented by being coupled to an apparatus, and may be implemented by a software. Accordingly, this disclosure may include an artificial intelligence insurance server for executing the method for providing an artificial intelligence insurance service described above. Furthermore, although the artificial intelligence insurance server according to various embodiments of this disclosure may be implemented by one apparatus, it may also be implemented by a plurality of apparatuses.

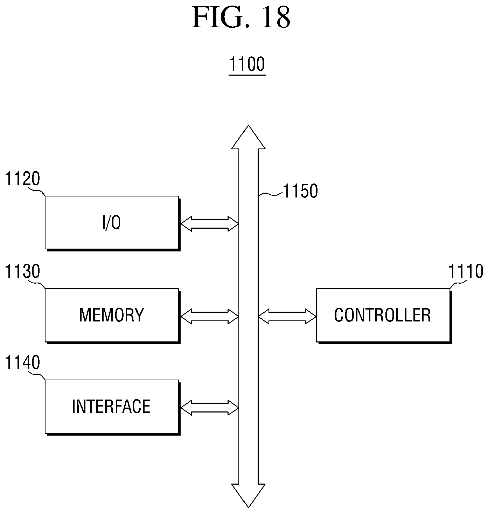

FIG. 18 is a block diagram of an electronic system for implementing an artificial intelligence insurance server and a method for providing an artificial intelligence insurance service according to some embodiments of the disclosure.

Referring to FIG. 18, the electronic system **1100** according to an embodiment of the disclosure may include a controller **1110,** an input/output device **1120** (I/O), a memory device **1130,** an interface **1140** and a bus **1150.** The controller **1110,** the input/output device **1120,** the memory device **1130,** and/or the interface **1140** may be coupled to each other through the bus **1150.** The bus **1150** corresponds to a path on which data is moved.

The controller **1110** may include at least one of a CPU (Central Processing Unit), MPU (Micro Processor Unit), MCU (Micro Controller Unit), GPU (Graphic Processing Unit), microprocessor, digital signal process, microcontroller, or logic elements that may perform similar functions thereof. The input/output device **1120** may include a keypad, keyboard and display device, or the like. The memory device **1130** may store data and/or commands, *etc.*

The interface **1140** may perform the function of transmitting data to a communication network or receiving data from a communication network. The interface **1140** may be wired or wireless. For example, the interface **1140** may include an antenna or a wired/wireless transceiver, *etc.* Although not shown, the electronic system **1100** may further include a highspeed DRAM and/or SRAM, *etc.* as an operation memory to enhance the operation of the controller. The fin field-effect transistor according to the embodiments of the disclosure may be provided within the memory device **1130** or provided as a part of the controller **1110** and the input/output device **1200** (I/O), or the like.

The electronic system **1100** may be applied to a personal digital assistant (PDA), a portable computer, a web tablet, a wireless phone, a mobile phone, a digital music player, a memory card, or any electronic product that can transmit and/or receive information in a wireless environment.

Although described by the embodiments of the disclosure in reference to the accompanying drawings, those of ordinary skill in the technical field to which the disclosure pertains would understand that the disclosure could be implemented in other specific forms without modifying the technical concept or essential features. Therefore, it should be understood that the embodiments described above are exemplary in all aspects and are not limiting.

## Claims

1. An artificial intelligence insurance server comprising:
a receiving unit for receiving input data including medical data of an insurance user;
a comparison data selection unit for selecting comparison data based on the input data and deriving first analysis data by analyzing the comparison data;
an artificial intelligence analysis unit for obtaining second analysis data derived by performing artificial intelligence analysis on the input data;
a comparison unit for comparing the first and the second analysis data, and generating final analysis data using the first and the second analysis data; and
a decision-making unit for making at least one insurance-related decision using the final analysis data.

2. The artificial intelligence insurance server according to claim 1,
wherein the receiving unit determines whether the input data is sufficient, and when the input data is sufficient, transmits a progress signal to the comparison data selection unit.

3. The artificial intelligence insurance server according to claim 1,
wherein the medical data includes at least one of an X-ray, CT (Computed Tomography), a mammography image, an MRI (Magnetic Resonance Imaging) image, an ultrasound image, a pathology slide image, a tissue image, genomic data including multi-omics data, biological data, a medical report, or a personal health record (PHR).

4. The artificial intelligence insurance server according to claim 1,
wherein the comparison data includes cohort data of the insurance user selected from the input data.

5. The artificial intelligence insurance server according to claim 4,
wherein the input data includes at least one of demographic information, or information on factors causing accidents or diseases of the insurance user, and
wherein the cohort data is selected from at least one of the demographic information, or the information on factors causing accidents or diseases.

6. The artificial intelligence insurance server according to claim 4,
wherein the cohort data is selected as data of a combination that is similar to the combination of the input data.

7. The artificial intelligence insurance server according to claim 1,
wherein the comparison data includes past data of the insurance user.

8. The artificial intelligence insurance server according to claim 1,
wherein the comparison data selection unit receives diagnosis data including diagnosis result of the input data, and converts the diagnosis data to first analysis data.

9. The artificial intelligence insurance server according to claim 1,
wherein the artificial intelligence analysis unit receives second analysis data for which artificial intelligence analysis is performed on the input data.

10. The artificial intelligence insurance server according to claim 1,
wherein the decision-making unit:
makes at least one decision on insurance product recommendation, underwriting or insurance premium discount calculation if the insurance user has not subscribed to insurance, and
makes at least one decision on health care, insurance benefits payout or additional insurance product recommendation if the insurance user has subscribed to insurance.

11. The artificial intelligence insurance server according to claim 1,
wherein the decision-making unit makes at least one decision on insurance benefits payout,
wherein the comparison unit:
determines whether a first additional test is needed using the first analysis data, and
determines whether a second additional test is needed using the second analysis data, and
wherein the decision-making unit requests the insurance user for an additional test if at least one of the first or the second additional test is determined to be needed.

12. The artificial intelligence insurance server according to claim 11,
wherein the receiving unit receives whether an additional test is carried out from the insurance user, and transmits whether the additional test is carried out to the decision-making unit, and
wherein the decision-making unit reduces insurance benefits of diagnosis for further diagnosis of the insurance user, if the second additional test is determined to be needed and the additional test is not carried out.

13. The artificial intelligence insurance server according to claim 11,
wherein the receiving unit receives whether an additional test is carried out from the insurance user, and transmits whether the additional test is carried out to the decision-making unit, and
wherein the decision-making unit:
determines whether an early diagnosis is made if the first additional test is determined not to be needed, the second additional test is determined to be needed, and the additional test is carried out, and
pays raised amount of insurance benefits if it is determined that the early diagnosis is made.

14. The artificial intelligence insurance server according to claim 1,
wherein the comparison data selection unit requests comparison data from a database, and
wherein the receiving unit receives the comparison data from the database, and transmits the comparison data to the comparison data selection unit.

15. A method for providing an artificial intelligence insurance service comprising:
receiving input data including medical data of an insurance user,
selecting comparison data based on the input data,
obtaining first analysis data for the comparison data,
obtaining second analysis data for the input data,
comparing the first and the second analysis data, and generating final analysis data using the first and the second analysis data, and
making at least one decision using the final analysis data.

16. The method for providing an artificial intelligence insurance service according to claim 15,
wherein the obtaining first analysis data for the comparison data includes obtaining first analysis data by performing artificial intelligence analysis on the comparison data.

17. The method for providing an artificial intelligence insurance service according to claim 15,
wherein the obtaining first analysis data for the comparison data includes:
receiving diagnosis data of medical staff for the comparison data, and
obtaining the first analysis data by converting the diagnosis data.

18. The method for providing an artificial intelligence insurance service according to claim 15,
wherein the input data includes at least one of demographic information, or information on factors causing accidents or diseases of the insurance user.

19. The method for providing an artificial intelligence insurance service according to claim 18,
wherein the comparison data includes cohort data selected from at least one of the demographic information, or the information on factors causing accidents or diseases.

20. The method for providing an artificial intelligence insurance service according to claim 15,
wherein the obtaining second analysis data includes:
selecting a corresponding portion of the input data that corresponds to the comparison data, and
obtaining the second analysis data by analyzing the corresponding portion.

21. The method for providing an artificial intelligence insurance service according to claim 15,
wherein the making at least one decision:
includes at least one of insurance product recommendation, underwriting or insurance premium discount calculation if the insurance user has not subscribed to insurance, and
includes at least one of health care, insurance benefits payout or additional insurance product recommendation if the insurance user has subscribed to insurance.

22. The method for providing an artificial intelligence insurance service according to claim 15,
wherein the making at least one decision includes insurance benefits payout, and wherein the insurance benefits payout includes:
determining whether a first additional test is needed using the first analysis data,
determining whether a second additional test is needed using the second analysis data, and
requesting the insurance user for an additional test if at least one of the first or the second additional test is determined to be needed.

23. The method for providing an artificial intelligence insurance service according to claim 22,
wherein the insurance benefits payout includes reducing insurance benefits of diagnosis for further diagnosis of the insurance user if the second additional test is determined to be needed, and the additional test is not carried out.

24. The method for providing an artificial intelligence insurance service according to claim 22,
wherein the insurance benefits payout includes:
determining whether an early diagnosis is made, if the first additional test is determined not to be needed, the second additional test is determined to be needed, and the additional test is carried out, and
paying raised amount of insurance benefits if the early diagnosis is made.

25. A method for providing an artificial intelligence insurance service comprising:
obtaining input data including at least one medical data of an insurance user;
performing artificial intelligence analysis on the medical data; and
providing information on at least one of underwriting, recommended insurance product, predicted insurance premium, or whether an additional test is needed for the insurance user based on the artificial intelligence analysis result.

26. The method for providing an artificial intelligence insurance service according to claim 25,
wherein the input data includes at least one of demographic information, or information on factors causing accidents or diseases of the insurance user.

27. The method for providing an artificial intelligence insurance service according to claim 26,
wherein the providing is providing whether an additional test is needed, and
wherein the providing whether an additional test is needed includes providing information on the additional test and information on disadvantages due to not implementing an additional test if the additional test is determined to be needed.
